# EUROPEAN PATENT SPECIFICATION

(11) **EP 1 075 264 B1**
(45) Date of publication and mention of the grant of the patent: **01.12.2004**
(21) Application number: 99917309.9
(22) Date of filing: 14.04.1999
(51) Int. Cl.: A61K 31/535, A61P 25/18, A61P 25/22, A61P 25/24

(54) **NEW DRUG COMBINATIONS OF REBOXETINE AND PINDOLOL**
NEUE ARZNEIMITTELKOMBINATIONEN AUS REBOXETIN UND PINDOLOL
NOUVELLES COMBINAISON MEDICAMENTEUSES DE LA REBOXETINE, ET DE PINDOLOL

(30) Priority: 08.05.1998 US 84860 P
(43) Date of publication of application: 14.02.2001
(73) Proprietor: PHARMACIA & UPJOHN COMPANY, Kalamazoo, Michigan 49001 (US)
(72) Inventor: TAYLOR, Duncan, Paul, Kalamazoo, MI 49009 (US)
(74) Representative: Duncan, Garreth Andrew
(86) International application number: PCT/US1999/006523
(87) International publication number: WO 1999/058130

(56) References cited:
- EP-A- 0 687 472
- EP-A- 0 759 299
- REDROBE ET AL: "The Role of 5-HT1A and 5-HT1B Receptors in Antidepressant Drug Actions in the Mouse Forced Swimming Test" EUROPEAN JOURNAL OF PHARMACOLOGY, vol. 318, no. 2/3, 30 December 1996 (1996-12-30), pages 213-220, XP002111616
- BLIER ET AL: "Selective Activation of Postsynaptic 5-HT1A Receptors Induces Rapid Antidepressant Response" NEUROPSYCHOPHARMACOLOGY, vol. 16, no. 5, 1997, pages 333-338, XP002111617
- MORENO ET AL: "Pindolol Augmentation of Treatment-Resistant Depressed Patients" JOURNAL OF CLINICAL PSYCHIATRY, vol. 58, no. 10, 1997, pages 437-439, XP002111618
- DEVANE: "Differential Pharmacology of Newer Antidepressants" THE JOURNAL OF CLINICAL PSYCHIATRY, vol. 59, no. S20, 1998, pages 85-93, XP002111619

## Description

### Field of the Invention

This invention describes new products that should provide for a fast acting rapid onset of relief from several nervous system disorders, and it involves the administration of the drug reboxetine in combination with the drug pindolol.

### Background

The introduction of tricyclic antidepressants in the early 1960s has provided a major advance in the treatment of neuropsychiatric disorders. Reactive and endogenous depressions, diagnoses formerly carrying grave prognostic implications, have become, with the introduction of the tricyclics, manageable disorders with a much smaller toll on the patient and the society as a whole.

The early tricyclic compounds were reuptake inhibitors of all the catecholamines released in the synaptic cleft, thus resulting in prolongation and enhancement of the dopamine (DA), noradrenaline (NA) and serotonin (5-hydroxytryptamine = 5-HT) action. Lack of selectivity also causes undesired side effects particularly on the acetylcholine (especially the muscarinic component), and histamine mediated neurotransmission.

Because of these unwanted pharmacodynamic activities, cognitive impairment, sedation, urinary and gastrointestinal tract disturbances, increased intraocular pressure were limiting factors in the clinical use of these compounds and often required discontinuation of treatment. Of utmost concern were also the cardiac toxic effects and the proconvulsant activity of this group of drugs.

More recently, selective reuptake inhibitors for serotonin (SSRI) have been introduced with definite advantages in regard to fewer side effects without loss of efficacy.

### Summary of the Invention

Here we present the surprising finding that when the drug pindolol is given to a patient concurrently with a drug from a new category of antidepressants, a so called noradrenaline (NA) reuptake inhibitor (NARI), the combination of drugs act with surprising speed in relieving the symptoms of depression and it may be used for treating the symptoms of other central nervous system disorders including. but not only, general anxiety. Addictive Disorders. attention deficit hyperactivity disorder (ADHD), anxiety disorders such as obsessive compulsive disorders (OCD), panic disorders (PD), social phobia (SP) and the like.

One particular NARI that is preferred is reboxetine. Reboxetine is the generic name of the pharmaceutical substance with the chemical name of 2-(I-((2-ethoxyphenoxy)benzyl)morpholine, and its pharmaceutically acceptable salts. Reboxetine can be a free base, or it can include reboxetine methanesulfonate (also called reboxetine mesylate) or any other pharmaceutically acceptable salt that does not significantly affect the pharmaceutical activity of the substance.

The chemical name of pindolol is 1-(1H-Indol-4-yloxy)-3-[(1-methylethyl)amino]-2-propanol; 4-[2-hydroxy-3-(isopropylamino)-propoxy]indole: pinodolol. Pindolol is described in US patent no. 3,471,515, and process steps are described in Swiss patents 469,002 and 472,404, assigned to the Sandoz Company, now the Novartis company. It has the trade name VISKEN®.

The present invention provides for the dosing of both reboxetine and pindolol, concurrently. The dosages for reboxetine and pindolol can be measured separately. The two drugs can be given as a single combined dose or given separately. They may be given at the same or at different times as long as both drugs are in the patient at one time over a 24 hour period. The two drugs will preferably be given to the patient, concomitantly, concurrently, at or about the same time, within about 5, 10, or 30 minutes, or they may be given within 1, 2, 3, 4, 5, 6, 8, 10, 12, 18 or about 24 hours, or fractions of minutes or of hours of each other. Concomitant or concurrent administration means the patient takes one drug within about 5 minutes of taking the other drug. Because the goal here is to provide rapid symptomatic relief to the patient, in most cases when treatment is started the two drugs would be administered to the patient close in time and typically concomitantly; thereafter, the timing of each drug's administration may not be as important.

A preferred dose range of reboxetine is 4 to 10 mg pcr patient per day and the more preferred dose is 6 to 8 mg or 8 to 10 mg per patient daily, depending upon the patient, delivered twice a day (b.i.d.). The reboxetine should be given to a patient concurrently with pindolol.

A preferred dose range of pindolol is 10-60 mg per patient per day and the more preferred dose is about 10 mg per patient daily, depending upon the patient, delivered twice a day (b.i.d.). Preferably the pindolol should be given concurrently with reboxetine as described above.

### Additional Description of the Invention and Description of the Preferred Embodiment(s)

Reboxetine is the generic name of the pharmaceutical substance with the chemical name of 2-(I-((2-ethoxyphenoxy)benzyl)-morpholine, and its pharmaceutically acceptable salts. Reboxetine can be a free base, or it can include reboxetine methanesulfonate (also called reboxetine mesylate) or any other pharmaceutically acceptable salt that does not significantly affect the pharmaceutical activity of the substance. Reboxetine and a method of synthesis are described in U.S. 4,229,449, issued 21 Oct. 1980, Melloni et. al., methods of preparation are described in US 5,068,433, issued 26 Nov. 1991, Melloni et. al. and in US 5,391,735, issued 21 Feb. 1995. Reboxetine may also be known under the trade name of EDRONAX™.

The pharmaceutical compositions and methods of administration described in US 4,229,449 at col. 18, lines 33-66. Twice a day dosing is preferred with current formulations.

Reboxetine acts as an antidepressant. Antidepressants are frequently grouped into categories or "generations." The first generation of antidepressants were usually tricyclic antidepressants such as maprotiline that affected various neurotransmitter systems and are associated with many undesirable side effects. The second generation of antidepressants. such as mianserine, mirtrazapine and trazodone are largely devoid of anticholinergic action and their adrenolytic and antihistaminic effects are weaker. These are contrasted with the third generation of antidepressants (e.g. SSRI, ipsapirone, viloxazine, reboxetine, bupropione) that mediate only one of the three main neurotransmitter system for depression (5-HT, noradrenaline, dopamine) and they do not affect muscarine, histamine and adrenergic cerebral systems. Svestka, J. "Antidepressives of the 3rd. 4th and 5th generation." Cesk-Psychiatr. 1994 Feb; 90(1):3-19. (Czech).

Reboxetine, however, does not act like most antidepressants. Unlike tricyclic antidepressants and even selective serotonin reuptake inhibitors (SSRIs), reboxetine is ineffective in the 8-OH-DPAT hypothermia test, indicating that reboxetine is not a selective serotonin reuptake inhibitor rather it is selective for the noradrenergic system. Thus, reboxetine is not an SSRI, rather it is considered a novel, selective, noradrenaline-reuptake inhibitor (NARI). Leonard-BE, "Noradrenaline in basic models of depression." *European-Neuropsychopharmacol*. 1997 Apr; 7 Suppl 1: S11-6; discussion S71-3. Unlike most drugs, reboxetine is a highly selective norepinephrine uptake inhibitor, with only marginal serotonin and no dopamine uptake inhibitory activity. The compound displays only weak or no anti-cholinergic activity in different animal models and is devoid of monoamine oxidase (MAO) inhibitory activity.

Reboxetine is highly potent and fast acting. Our investigations indicate reboxetine has potent antireserpine activity and combines the inhibitory properties of classical tricyclic antidepressants on the reuptake of noradrenaline with an ability to desensitize ϑ-adrenergic receptor function without showing any appreciable interaction with muscarinic cholinergic and I-adrenerigic receptors. Moreover, reboxetine shows less vagolytic activity than other tricyclic antidepressants.

In spite of the inherently fast action of reboxetine there is still a "lag" or delay from the time of administration of the drug until the time the drug provides symptomatic relief to the patient. The treatments described here decrease that lag time. A period of days and especially weeks between the administration of a drug and its effect in relieving depression can be devastating to a patient. The patient may conclude the drug is not effective and stop taking the drug, thus a quick onset of activity is critically important for treatments of this type. We have discovered that the combination of pinodolol and reboxetine provides highly effective relief of psychiatric disorders with a minimal delay in onset of activity.

Pindolol is the generic name for 1-(1H-Indol-4-yloxy)-3-[(1-methylethyl)amino]-2-propanol; 4-[2-hydroxy-3-(isopropylamino)-propoxy]indole; prinodolol. Pindolol is described in US patent no. 3,471,515 and process steps are described in Swiss patents 469,002 and 472,404, assigned to the Sandoz Company, now the Novartis company. It has the trade name VISKEN®.

The dosage used to treat all of the disorders described here may be found above and below. Reboxetine is well tolerated and has a wide safety range, it can be administered in a dose range of active ingredient from about 1 to over 40 mg/kg. It is more commonly provided in dosages of from 1 to 20 mg per patient per day. Pindolol is also fairly safe although it is contraindicated for patients with bronchial asthmas, cardiac failure, heart block and severe bradycardia. Other adverse reactions are possible. Pindolol dosages in the range of 5 to 60 mg daily can be effective. Both compounds may be administered by any suitable method including a convenient oral dosage form. A preferred method is oral dosing twice a day. The preferred dose range of reboxetine is 4 to 20 and more preferably 4 to 10 mg per patient per day and the preferred dose range of pindolol is 10 - 20 mg per patient per day. When starting medication the more preferred dose of reboxetine is 6 to 8 mg or 8 to 10 mg and pindolol is 10 mg per patient daily, depending upon the patient, delivered twice a day (b.i.d.). It can also be given at dosages of 2, 4, 6, 8, 10 or 12 mg/patient per day or fractions thereof: For example, suitable administrations could be 4 mg of reboxetine and 5 mg of pindolol in the morning and 2 or 4 mg of Rebozetine and 5 mg of pindolol in the evening. A skilled practitioner would be expected to determine the precise level of dosing. The idea dosing would be routinely determined by an evaluation of the patient and the needs of the patient.
This patent application describes the treatment of numerous conditions, disorders, diseases, and disease symptoms with the combination of drugs described herein, in addition to the conditions, disorders, diseases, and disease symptoms described above, the following may also be treated with these drugs: Addictive Disorders, Psychoactive Substance Use Disorders, Nicotine Addition or Tobacco Addiction (with a result of Smoking Cessation or a decrease in smoking) and Attention Deficit Hyperactivity Disorder (ADHD). This patent application also describes the treatment of Obsessive Compulsive Disorders (OCD), and Panic Disorder (PD), comprising administering a therapeutically effective, nontoxic dose of the drugs described herein and derivatives and or pharmaceutically acceptable salts thereof to a patient

Addictive Disorders and Psychoactive Substance Use Disorders, such as Intoxication disorders, Inhalation disorders, Alcohol addiction, Tobacco Addiction and or Nicotine Addiction. Tobacco and Nicotine addiction would be treated with the goal of achieving either Smoking Cessation or Smoking Reductions.

Addictive Disorders, Alcohol and Other Psychoactive Substance Use Disorders, disorders related to Intoxication and Inhalants and especially Tobacco Addiction or Nicotine Addiction, may be treated with the drugs described herein. Tobacco Addiction or Nicotine Addiction would be treated with the drugs described herein in order to achieve smoking/chewing cessation or smoking/chewing reduction. General descriptions of Addictive Disorders, including disorders related to Intoxication and Inhalants and Tobacco Addiction or Nicotine Addiction may be found in many standard sources, such as, The American Psychiatric Press Textbook of Psychiatry, Second Edition, Edited by Robert E. Hales, Stuart C. Yudofsky, and John A. Talbott, copyright 1994, especially pp. 401 et. seq., section on "Nicotine" incorporated by reference. Another of many texts is the Manual of Psychiatric Therapeutics, Second Edition, edited by Richard I. Shader, especially pp. 85 from Chapter 11 (Hypnosis).

The treatment of Alcohol and Other Psychoactive Substance Use Disorders, such as disorders related to Intoxication and Inhalants and Tobacco Addiction or Nicotine Addiction but especially Tobacco Addiction involves the administration of the drugs described herein in a manner and form that provide a reduction in the symptoms of the disease. Tobacco Addiction or Nicotine Addiction in particular would be treated to achieve a reduction or cessation of smoking or chewing of nicotine containing materials by a patient. Cessation or a reduction in smoking or chewing of addictive or psychoactive substances involves the administration of the drugs described herein in a manner and form that provide a reduction in the symptoms of the disease, or with Tobacco or Nicotine with a reduction in the amount smoked or chewed.. See the general description above for administration of Reboxetine.

### Attention Deficit Hyperactivity Disorder (ADHD)

ADHD is a condition or disease state that may be treated with the drugs described herein. General descriptions of ADHD, may be found in many standard sources, such as, The American Psychiatric Press Textbook of Psychiatry, Second Edition, Edited by Robert E. Hales, Stuart C. Yudofsky, and John A. Talbott, copyright 1994, especially pp. 741 et. al., section on "ADHD,". Another of many texts is the Manual of Psychiatric Therapeutics, Second Edition, edited by Richard I. Shader, incorporated by reference, especially Chapter 18, Attention-Deficit hyperactivity Disorder, and pp. 172 et. seq..

The treatment of Attention Deficit Hyperactivity Disorder in children and adults involves the administration of the drugs described herein in a manner and form that provide a reduction in the symptoms of the disease. A child or young adult may require a smaller dosage depending upon the size, age, condition of the patient. See general description above for administration of the drugs described herein.

### Obsessive Compulsive Disorders (OCD)

Obsessive Compulsive Disorder is a condition or state of anxiety that may be treated with reboxetine. General descriptions of OCD, may be found in many standard sources, such as, The American Psychiatric Press Textbook of Psychiatry, Second Edition, Edited by Robert E. Hales, Stuart C. Yudofsky, and John A. Talbott, copyright 1994, especially the chapter on "Anxiety Disorders". Another of many texts is the Manual of Psychiatric Therapeutics, Second Edition, edited by Richard I. Shader, especially Chapter 5, Obsessions and Compulsions, more particularly, Section III of that chapter, "OCD" pp. 36 *et*. *seq*..

The treatment of Obsessive Compulsive Disorders (OCD) involves the administration of reboxetine in a manner and form that provide a reduction in the symptoms of the disease. See general description above for administration of reboxetine.

The following study shows the therapeutic effectiveness of using reboxetine in doses varying from 6 to 8 mg to treat OCD. This study is provided to illustrate the usefulness of using reboxetine as a treatment for OCD and the invention described herein should not be considered limited by this example.

In a trial involving 10 patients with a DSM-III-R diagnosis of Obsessive Compulsive Disorder who were all treated with reboxetine for a period of 3 to 4 weeks with the dose for the first week at 6 mg (4 mg in a.m. and 2 mg in p.m.) with the dose increasing in the second week to 8 mg (4 mg b.i.d.). At CGI last assessment, one patient was judged very much improved, 4 were judged much improved, 2 minimally improved. while 3 were unchanged. Of the patients who did respond they had a decrease of the obsessive-compulsive symptomatology, as measured by the CPRS-OC rating scale, of more than 30 and as much as 73%.

### Panic Disorder (PD)

Panic Disorder is a condition or state of anxiety that may be treated with reboxetine. General descriptions of PD, may be found in many standard sources, such as, The American Psychiatric Press Textbook of Psychiatry, Second Edition, Edited by Robert E. Hales, Stuart C. Yudofsky, and John A. Talbott, copyright 1994, especially the chapter on "Anxiety Disorders," another of many texts is the Manual of Psychiatric Therapeutics, Second Edition, edited by Richard I. Shader, especially Chapter 25. "Approaches to the Treatment of Anxiety States".

The treatment of Panic Disorder involves the administration of the drugs described herein in a manner and form that provide a reduction in the symptoms of the disease. See general description above.

## Claims

1. A product comprising pindolol or a derivative or pharmaceutically acceptable salt thereof and reboxetine or a pharmaceutically acceptable salt thereof, as a combined preparation for simultaneous, separate or sequential use in the treatment of a condition selected from depression, general anxiety disorders (GADs), Addictive Disorders, attention deficit hyperactivity disorder (ADHD) and anxiety disorders such as obsessive compulsive disorder (OCD), panic disorder (PD) or social phobia (SP).

2. A product according to claim 1, wherein the condition is depression.

3. A product according to claim 1, wherein the condition is a GAD.

4. A product according to claim 1, wherein the condition is an addictive disorder.

5. A product according to claim 1, wherein the condition is ADHD.

6. A product according to claim 1, wherein the condition is OCD.

7. A product according to claim 1, wherein the condition is PD.

8. A product according to claim 1, wherein the condition is SP.

9. A product according to any preceding claim, which comprises dosages sufficient to provide a reboxetine dose range of 4 to 10 mg per patient per day and a pindolol dose range of 10 to 20 mg per patient per day, delivered twice per day.

10. A product according to claim 9, wherein the reboxetine dose range is 6 to 8 mg per patient per day and the pindolol dose range is 10 to 16 mg per patient per day, delivered twice per day.

11. A product according to claim 9, which is a single dosage form.

12. A product according to claim 11, which is a tablet, capsule or caplet.

13. A product according to claim 11 or claim 12, which comprises 5 mg reboxetine and 7.5 mg pindolol.

14. The use of reboxetine or a derivative or pharmaceutically acceptable salt thereof and pindolol or a derivative or pharmaceutically acceptable sal thereof for the manufacture of a medicament for use in the treatment of a condition as defined in an y of claims 1 to 8.

## Patentansprüche

1. Ein Produkt, welches Pindolol oder eines seiner Derivate oder pharmazeutisch annehmbaren Salze und Reboxetin oder eines seiner pharmazeutisch annehmbaren Salze umfasst, als Kombinationspräparat für die gleichzeitige, getrennte oder sequenzielle Verwendung bei der Behandlung eines Zustandes, ausgewählt aus Depression, allgemeine Angststörungen (GADs), suchtbedingte Störungen, Aufmerksamkeitsdefizit-Syndrom mit Hyperaktivität (ADHD) und Angststörungen, wie z. B. Zwangsneurose (OCD), Panik-Störung (PD) oder soziale Phobie (SP).

2. Ein Produkt gemäß Anspruch 1, wobei der Zustand eine Depression ist.

3. Ein Produkt gemäß Anspruch 1, wobei der Zustand eine GAD ist.

4. Ein Produkt gemäß Anspruch 1, wobei der Zustand eine suchtbedingte Störung ist.

5. Ein Produkt gemäß Anspruch 1, wobei der Zustand ADHD ist.

6. Ein Produkt gemäß Anspruch 1, wobei der Zustand OCD ist.

7. Ein Produkt gemäß Anspruch 1, wobei der Zustand PD ist.

8. Ein Produkt gemäß Anspruch 1, wobei der Zustand SP ist.

9. Ein Produkt gemäß irgendeinem der vorhergehenden Ansprüche, welches Dosierungen enthält, die ausreichen, um einen Reboxetin-Dosisbereich von 4 bis 10 mg pro Patient und Tag und eine Pindolol-Dosisbereich von 10 bis 20 mg pro Patient und Tag bei täglich zweimaliger Verabreichung zur Verfügung zu stellen.

10. Ein Produkt gemäß Anspruch 9, bei dem der Reboxetin-Dosierungsbereich 6 bis 8 mg pro Patient und Tag und der Pindolol-Dosierungsbereich 10 bis 16 mg pro Patient und Tag bei täglicher zweimaliger Verabreichung beträgt.

11. Ein Produkt gemäß Anspruch 9, als Einzeldosierungsform.

12. Ein Produkt gemäß Anspruch 11, in Form einer Tablette, einer Kapsel oder eines Caplets.

13. Ein Produkt gemäß Anspruch 11 oder Anspruch 12, welches 5 mg Reboxetin und 7,5 mg Pindolol unfasst.

14. Die Verwendung von Reboxetin oder eines seiner Derivate oder pharmazeutisch annehmbaren Salze und Pindolol oder eines seiner Derivate oder pharmazeutisch annehmbaren Salze zur Herstellung eines Medikaments für den Einsatz bei der Behandlung eines in irgendeinem der Ansprüche 1 bis 8 definierten Zustandes.

## Revendications

1. Produit comprenant du pindodol, ou un dérivé ou sel pharmaceutiquement acceptable de celui-ci, et de la réboxétine, ou un dérivé ou sel pharmaceutiquement acceptable de celle-ci, sous la forme d'une préparation combinée pour une utilisation simultanée, séparée ou successive, dans le traitement d'un état sélectionné entre la dépression, les troubles anxieux généralisés (GAD), les troubles de dépendance, les troubles d'hyperactivité avec déficit d'attention (THADA) et les troubles anxieux tels que les troubles obsessionnels compulsifs (TOC), les troubles panique (TP) ou la phobie sociale (PS).

2. Produit selon la revendication 1, dans lequel l'état est la dépression.

3. Produit selon la revendication 1, dans lequel l'état est un GAD.

4. Produit selon la revendication 1, dans lequel l'état est un trouble de dépendance.

5. Produit selon la revendication 1, dans lequel l'état est un THADA.

6. Produit selon la revendication 1, dans lequel l'état est un TOC.

7. Produit selon la revendication 1, dans lequel l'état est un DP.

8. Produit selon la revendication 1, dans lequel l'état est la PS.

9. Produit selon l'une quelconque des revendications précédentes, qui comprend des posologies suffisantes pour offrir une gamme de doses de réboxétine allant de 4 à 10 mg par patient et par jour et une gamme de doses de pindodol allant de 10 à 20 mg par patient et par jour, délivrées deux fois par jour.

10. Produit selon la revendication 9, dans lequel la gamme de doses de réboxétine va de 6 à 8 mg par patient et par jour et la gamme de doses de pindodol va de 10 à 16 mg par patient et par jour, délivrées deux fois par jour.

11. Produit selon la revendication 9, qui est une forme dosée unique.

12. Produit selon la revendication 11, qui est un comprimé, une capsule ou un caplet.

13. Produit selon la revendication 11 ou la revendication 12, qui comprend 5 mg de réboxétine et 7,5 mg de pindodol.

14. Utilisation de la réboxétine, ou d'un dérivé ou sel pharmaceutiquement acceptable de celle-ci, et du pindodol, ou d'un dérivé ou sel pharmaceutiquement acceptable de celui-ci, pour la fabrication d'un médicament destiné au traitement d'un état tel que défini dans l'une quelconque des revendications 1 à 8.
